**Europäisches Patentamt**

**European Patent Office**

**Office européen des brevets**

(19)

(11) Veröffentlichungsnummer: **0 064 693**
**B1**

(12) # EUROPÄISCHE PATENTSCHRIFT

(45) Veröffentlichungstag der Patentschrift:
**10.04.85**

(51) Int. Cl.⁴: **C 07 C 143/675**

(21) Anmeldenummer: **82103700.9**

(22) Anmeldetag: **30.04.82**

(54) Verfahren zur Herstellung von 1-Benzoylamino-8-hydroxy-naphthalin-4,6-disulfonsäure (Benzoyl-K-Säure).

(30) Priorität: **13.05.81 DE 3118903**

(43) Veröffentlichungstag der Anmeldung:
**17.11.82 Patentblatt 82/46**

(45) Bekanntmachung des Hinweises auf die Patenterteilung:
**10.04.85 Patentblatt 85/15**

(84) Benannte Vertragsstaaten:
**CH DE FR GB IT LI**

(56) Entgegenhaltungen:
**DE - A - 2 806 951**
**DE - A - 2 843 680**

**ULLMANNS ENCYCLOPÄDIE DER TECHNISCHEN CHEMIE, 4. Auflage, Band 17, 1979 VERLAG CHEMIE, Weinheim, New York, Seite 105**

(73) Patentinhaber: **BAYER AG, Konzernverwaltung RP Patentabteilung, D-5090 Leverkusen 1 Bayerwerk (DE)**

(72) Erfinder: **Hammerschmidt, Erich, Dr.,**
**Schützenstrasse 25, D-5060 Bergisch Gladbach 2 (DE)**
Erfinder: **Behre, Horst, Dr., Zur alten Linde 12,**
**D-5068 Odenthal (DE)**
Erfinder: **Krüger, Bruno, Dr., Am Haferkamp 1,**
**D-5000 Köln 80 (DE)**
Erfinder: **Winkler, Adolf, Dr., Christian-Hess-Strasse 69,**
**D-5090 Leverkusen 1 (DE)**

**Beschreibung**

Die Erfindung betrifft ein verbessertes Verfahren zur Herstellung von 1-Benzoylamino-8-hydroxy-naphthalin-4.6-disulfonsäure (Benzoyl-K-Säure) in Form ihrer Alkalisalze.

Benzoyl-K-Säure ist ein wichtiges Zwischenprodukt zur Herstellung von Farbstoffen (s. Ullmanns Enzyklopädie der technischen Chemie, 4. Auflage, Band 17, S. 107). Benzoyl-K-Säure (Dialkalisalz) wird bislang durch Umsetzung von 1-Amino-8-naphthol-4.6-disulfonsäure (K-Säure) mit Benzoylchlorid in sodaalkalischer Lösung bei 35 bis 40°C, Spaltung der bei der Umsetzung entstehenden O-Benzoylverbindung durch Erwärmen der Reaktionsmischung auf 90°C und anschliessendes Ausfällen der Benzoyl-K-Säure durch Ansäuern der Reaktionsmischung hergestellt (s. Ullmanns Enzyklopädie der technischen Chemie, loc. cit.). Nachteilig an diesem Verfahren ist, dass es als Ausgangsverbindung reine K-Säure erfordert. Reine K-Säure ist nur in schlechten Ausbeuten erhältlich, weil bei der Abscheidung und Isolierung von K-Säure aus K-Säure enthaltenden Reaktionsgemischen und nachfolgender Reinigung der isolierten K-Säure erhebliche Verluste an K-Säure auftreten.

Es wurde nun gefunden, dass es nicht erforderlich ist, für die Herstellung von Benzoyl-K-Säure von reiner K-Säure auszugehen, sondern dass man Benzoyl-K-Säure in unveränderter Reinheit aber in wesentlich verbesserter Ausbeute (bezogen auf Melansäure bzw. Naphthalin) herstellen kann, wenn man anstelle von reiner K-Säure K-Säure enthaltende Amino-naphthol-disulfonsäure-Isomerengemische benzoyliert. Es wurde nämlich überraschenderweise gefunden, dass die in diesen Gemischen enthaltenen anderen Isomeren das Ausfällen der Benzoyl-K-Säure dann nicht beeinträchtigen, wenn der Anteil der einzelnen Isomeren — mit Ausnahme von K-Säure — im Gesamtisomerengemisch, unabhängig von der Gesamtzahl der im Gemisch enthaltenen Isomeren, nicht mehr als 10 Gew.-% beträgt.

Die Erfindung betrifft daher ein Verfahren zur Herstellung von Benzoyl-K-Säure durch Umsetzung von K-Säure mit Benzoylierungsmitteln, das dadurch gekennzeichnet ist, dass man anstelle von reiner K-Säure Amino-naphthol-disulfonsäure-Isomerengemische einsetzt, die über 40 Gew.-% K-Säure enthalten, bezogen auf die Gesamtmenge an diazotierbaren Verbindungen im Isomerengemisch bei Zugrundelegung eines Molekulargewichtes von 319, und in denen der Anteil der einzelnen anderen Isomeren an der Gesamtmenge der diazotierbaren Verbindungen im Isomerengemisch nicht mehr als 10 Gew.-% beträgt.

Die erfindungsgemäss zu verwendenden Amino-naphthol-disulfonsäure-Isomerengemische enthalten vorzugsweise 50 bis 90 Gew.-% K-Säure, bezogen auf die Gesamtmenge an diazotierbaren Verbindungen in den Isomerengemischen bei Zugrundelegung eines Molekulargewichtes von 319. Besonders bevorzugt sind Amino-naphthol-disulfonsäure-Isomerengemische, die 55 bis 75 Gew.-% K-Säure, 0 bis 10 Gew.-% H-Säure und 2 bis 10 Gew.-% iso-K-Säure enthalten (Gew.-% bezogen auf die Gesamtmenge an diazotierbaren Verbindungen im Isomerengemisch bei Zugrundelegung eines Molekulargewichtes von 319).

Die erfindungsgemäss zu verwendenden Amino-naphthol-disulfonsäure-Isomerengemische können ausser K-Säure und den anderen Amino-naphthol-disulfonsäure-Isomeren, z.B. H-Säure und Iso-K-Säure, noch Nebenprodukte, Zersetzungsprodukte oder nicht-umgesetzte Ausgangsprodukte aus den der alkalischen Druckhydrolyse der Amino-naphthalin-trisulfonsäuregemische vorausgehenden Verfahrensstufen (Sulfonierung, Nitrierung, Reduktion) enthalten, vorausgesetzt, dass der Anteil der einzelnen, Aminogruppen aufweisenden Verbindungen an der Gesamtmenge der diazotierbaren Verbindungen im Isomerengemisch weniger als 10 Gew.-% beträgt. Als Nebenprodukte, Zersetzungsprodukte und nicht-umgesetzte Ausgangsprodukte seien beispielsweise genannt: Naphthalin-di-, -tri- und -tetrasulfonsäuren; Nitro-naphthalin-mono-, -di- und -trisulfonsäuren; Naphthylamin-mono-, -di- und -tri-sulfonsäure, z.B. 1-Amino-naphthalin-4.6- und -4.8-disulfonsäure oder 2-Amino-naphthalin-4.8-disulfonsäure; Naphthol-mono- und -di-sulfonsäuren, Dihydroxynaphthalin-mono- und -di-sulfonsäuren, weiterhin Dinaphthylsulfon-sulfonsäuren und deren Amino-, Nitro- und Hydroxy-Derivate sowie Oxidationsprodukte des Naphthalins und/oder der Naphthalinsulfonsäuren.

Solche erfindungsgemäss einzusetzenden, gegebenenfalls noch Neben-, Zersetzungs- oder Ausgangsprodukte aus vorausgehenden Verfahrensstufen enthaltende Amino-naphthol-disulfonsäure-Isomerengemische fallen beispielsweise bei der alkalischen Druckhydrolyse von 1-Naphthylamin-4.6.8-trisulfonsäure (Melansäure) oder von Melansäure-Isomerengemischen an (Melansäure-Isomerengemische = Naphthylamin-trisulfonsäure-Isomerengemische, die mindestens 40 Gew.-%, vorzugsweise 45 bis 75 Gew.-% Melansäure enthalten, bezogen auf die Gesamtmenge an diazotierbaren Verbindungen im Isomerengemisch bei Zugrundelegung eines Molekulargewichtes von 319. Die Herstellung von Melansäure-Isomerengemischen ist z.B. in FIAT Final Report, No. 1016, S. 42-44 beschrieben).

Falls die bei der alkalischen Druckhydrolyse der Melansäure-Isomerengemische anfallenden Hydrolysegemische mehr als 10 Gew.-% 1-Amino-8-hydroxy-naphthalin-3.6-disulfonsäure (H-Säure) enthalten, bezogen auf die Gesamtmenge an diazotierbaren Verbindungen im Hydrolysegemisch, wird aus diesen alkalischen Hydrolysegemischen, bevor sie für die Benzoylierung eingesetzt werden, durch Ansäuern auf einen pH-Wert von 0 bis 4, vorzugsweise 0,5 bis 2,5, und gegebenenfalls Verdünnen mit Wasser die H-Säure als Monoalkalisalz ausgefällt und abgetrennt. (Diese Abtrennung ist in der DE-OS 2 843 680 beschrieben).

Die erfindungsgemäss einzusetzenden Amino-naphthol-disulfonsäure-Isomerengemische werden in wässriger Lösung oder Suspension benzoyliert. Die Benzoylierung kann bei pH-Werten von 3 bis 10 vorgenommen werden. Vorzugsweise beträgt der pH-Wert der zu benzoylierenden wässrigen Lösungen oder Suspensionen 7 bis 10, insbesondere 8 bis 9. Zum Einstellen und Einhalten des pH-Wertes werden üblicherweise Alkalihydroxide oder Alkalicarbonate in fester Form oder in Form wässriger Lösungen verwendet. Bevorzugt werden Natriumcarbonat oder Natriumhydroxid eingesetzt.

Für die Benzoylierung der erfindungsgemäss einzusetzenden Amino-naphthol-disulfonsäure-Isomerengemische können alle zur N-Benzoylierung geeigneten Verbindungen verwendet werden. Bevorzugte Benzoylierungsmittel sind Benzoesäureanhydrid und Benzoylhalogenide; vorzugsweise wird Benzoylchlorid verwendet. Das Benzoylierungsmittel wird in einer solchen Menge eingesetzt, dass auf 1 Mol diazotierbare Verbindung in den Amino-naphthol-disulfonsäure-Isomerengemischen 1,0 bis 1,7 Mol, vorzugsweise 1,1 bis 1,4 Mol Benzoylierungsmittel entfallen.

Die erfindungsgemässe Umsetzung der Amino-naphthol-disulfonsäure-Isomerengemische mit dem Benzoylierungsmittel wird bei Temperaturen von 10 bis 100°C, vorzugsweise 25 bis 75°C, durchgeführt. Die Spaltung der O-Benzoylverbindung wird innerhalb derselben Temperaturbereiche, bevorzugt bei Temperaturen von 40 bis 95°C, vorgenommen.

Die Reaktionszeit hängt im wesentlichen von der Reaktionstemperatur und vom pH-Wert während der Reaktion ab. Mit steigender Temperatur und steigendem pH-Wert des Reaktionsgemisches verkürzt sich vor allem die für die Spaltung der O-Benzoylverbindung erforderliche Zeit.

Die Abscheidung der Benzoyl-K-Säure in Form ihrer Alkalisalze wird vorteilhaft bei pH-Werten < 7, vorzugsweise bei pH-Werten von 3 bis 6, insbesondere 4 bis 5, vorgenommen. Die Abscheidung lässt sich durch Einhaltung bestimmter Alkaliionen-Konzentrationen im Benzoylierungsgemisch verbessern. Diese Alkaliionen-Konzentrationen betragen etwa 2 bis 5 g Äquivalent/l Benzoylierungsgemisch. Falls die Benzoylierungsgemische die gewünschten Mengen an Alkaliionen nicht bereits enthalten, werden die gewünschten Alkaliionen-Konzentrationen durch Zugabe von im Benzoylierungsgemisch löslichen Alkalisalzen, vorzugsweise Natriumsalzen wie Natriumchlorid oder Natriumsulfat eingestellt.

Die Benzoyl-K-Säure (Alkalisalz) wird bei Temperaturen unter 60°C, üblicherweise 0 bis 60°C, vorzugsweise bei Temperaturen von 15 bis 45°C vom Benzoylierungsgemisch abgetrennt.

Das abgetrennte (abfiltrierte oder abgeschleuderte) Produkt wird mit Alkalisalzlösung gewaschen und anschliessend, gegebenenfalls im Vakuum, getrocknet. Durch Abstimmen von Alkaliionenkonzentrationen, pH-Wert und Isoliertemperatur lässt sich erreichen, dass allein das Alkalisalz der Benzoyl-K-Säure aus dem Benzoylierungsgemisch ausfällt, während die anderen im Benzoylierungsgemisch enthaltenen Verbindungen in Lösung bleiben.

Benzoyl-K-Säure wird nach dem erfindungsgemässen Verfahren in hoher Ausbeute, bezogen auf den K-Säure-Gehalt in den erfindungsgemäss zu verwendenden Amino-naphthol-disulfonsäure-Isomerengemischen, und in ausgezeichneter Reinheit erhalten. Dies ist deshalb überraschend, da in den erfindungsgemäss als Ausgangsverbindungen zu verwendenden Amino-naphthol-disulfonsäure-Isomerengemischen eine grosse Zahl chemisch sehr ähnlicher Verbindungen vorliegen, die in gleicher Weise wie K-Säure Benzoylderivate bilden, die in ihren physikalischen und chemischen Eigenschaften denen der Benzoyl-K-Säure sehr nahe kommen. Es war deshalb zu erwarten, dass sich Benzoyl-K-Säure aus solchen Benzoylierungsgemischen nicht in reiner Form und dazu in guten Ausbeuten würde abscheiden lassen.

Das erfindungsgemässe Verfahren bietet gegenüber der bekannten Benzoylierung von reiner K-Säure erhebliche Vorteile. Da beim erfindungsgemässen Verfahren die Verfahrensstufen Abscheiden, Isolieren und Reinigen der K-Säure entfallen, wird die bei der alkalischen Druckhydrolyse entstehende K-Säure vollständig für die Benzoylierung ausgenutzt und auf diese Weise eine verbesserte Ausbeute an Benzoyl-K-Säure, bezogen auf eingesetzte Melansäure bzw. auf ursprünglich eingesetztes Naphthalin, erzielt. Gegenüber den bislang erreichten Ausbeuten an Benzoyl-K-Säure wird eine relative Ausbeuteerhöhung um 8 bis 14% erreicht. Ausserdem führt das erfindungsgemässe Verfahren zu einer geringeren Belastung des Abwassers durch organischen Kohlenstoff. Ferner wird durch den Fortfall der K-Säure-Isolierung der Salz- und Säureverbrauch für das Abscheiden und damit die Menge der entsprechenden Verbindungen im Abwasser gegenüber den bekannten Verfahren um mehr als die Hälfte vermindert.

*Beispiel 1*

600 ml alkalische K-Säure-Lösung (erhalten durch alkalische Druckhydrolyse von Melansäure; Gehalt an diazotierbaren Verbindungen: 0,4 Mol; Gehalt an K-Säure: 0,34 Mol) werden durch Zugabe von 30%iger Salzsäure auf einen pH-Wert von 8,8 eingestellt und gleichzeitig mit Wasser auf ein Gesamtvolumen von 830 ml verdünnt. Diese Lösung wird bei 25 bis 45°C innerhalb von 2 Stunden mit 67,5 g (0,48 Mol) Benzoylchlorid versetzt und durch gleichzeitige Zugabe von etwa 10 ml 50%iger Natronlauge auf einem pH-Wert von 8,4 bis 8,8 gehalten. Die Reaktionsmischung wird 2 Stunden bei 40 bis 45°C unter Einhaltung des pH-Wertes gerührt, dann mit Natronlauge auf einen pH-Wert von 9,5 eingestellt und 1,5 Stunden unter Einhaltung dieses pH-Wertes auf 90 bis 95°C erwärmt.

Anschliessend wird die Reaktionsmischung mit Wasser auf 1700 ml verdünnt und bei 80 bis 90°C mit etwa 20 ml 30%iger Salzsäure auf einen pH-Wert von 6 und anschliessend mit 30 ml Essigsäure auf einen pH-Wert von 5 eingestellt. Beim langsamen Abkühlen auf 20°C fällt die Benzoyl-K-Säure aus. Sie wird abgesaugt und 2-mal mit je 100 ml halbgesättigter Kochsalzlösung gewaschen.

Die Zusammensetzung des abfiltrierten feuchten Produktes wird dünnschichtchromatographisch bestimmt. Die Ausbeute an reiner (100%iger) Benzoyl-K-Säure beträgt 137 g (= 95% der Theorie bezogen auf die in der alkalischen Hydrolyselösung enthaltene K-Säure bzw. 79,5% bezogen auf die in die Druckhydrolyse eingesetzte Melansäure). Die isolierte Benzoyl-K-Säure ist frei von H-Säure, K-Säure und Iso-K-Säure (1-Amino-6-hydroxy-naphthalin-4.8-disulfonsäure).

Wird anstelle der alkalischen K-Säure-Lösung eine dem K-Säure-Gehalt der Lösung äquivalente Menge reiner K-Säure benzoyliert, so beträgt die Ausbeute an Benzoyl-K-Säure nur 72% der Theorie, bezogen auf (die zur Herstellung der K-Säure verwendete) Melansäure.

*Beispiel 2*

Es werden 2504 g einer Amino-naphthol-disulfonsäure-Isomerengemisch-Lösung eingesetzt, die durch alkalische Druckhydrolyse eines Melansäure-Isomerengemisches und nachfolgendes Abtrennen der durch Ansäuern des alkalischen Hydrolysegemisches ausgefällten H-Säure erhalten wurde (Gehalt der Lösung an):

| | |
|---|---|
| diazotierbaren Verbindungen | 0,4 Mol |
| K-Säure | 0,27 Mol |
| H-Säure | 0,02 Mol |
| iso-K-Säure | 0,02 Mol |

Die Lösung enthält ausser K-, H- und Iso-K-Säure noch eine Vielzahl quantitativ nicht bestimmbarer Mengen an Neben-, Zersetzungs- und Oxidationsprodukten, die durch Trisulfierung von Naphthalin, anschliessende Nitrierung, Reduktion und alkalische Druckhydrolyse entstanden sind. Die Lösung wird durch Zugabe von Natronlauge auf einen pH-Wert von 8,8 eingestellt. Die Lösung wird bei 20°C unter Einhaltung eines pH-Wertes von 8,4 bis 8,8 mit 67,5 g (0,48 Mol) Benzoylchlorid versetzt. Die Reaktionsmischung wird unter Einhaltung des pH-Wertes zunächst 2 Stunden bei 45°C, dann 2 Stunden bei 70°C nachgerührt. Dann wird durch Zugabe von Natriumsulfat in der Reaktionslösung eine Natriumionenkonzentration von 3,7 g Äquivalenten/l eingestellt. Anschliessend wird durch Ansäuern des Reaktionsgemisches auf einen pH-Wert von 4,5 bis 5,0 mit (44 ml) 50%iger Schwefelsäure und langsames Abkühlen auf 40°C die Benzoyl-K-Säure abgeschieden. Sie wird abgesaugt und 2-mal mit je 100 ml halbgesättigter Natriumsulfat-Lösung gewaschen und anschliessend 24 Stunden bei 80°C im Vakuum (270 mbar) getrocknet.

Die Ausbeute an 100%iger Benzoyl-K-Säure beträgt 109 g ( = 95% der Theorie bezogen auf die in der Isomerengemischlösung enthaltene K-Säure bzw. 40% bezogen auf Naphthalin).

Die Zusammensetzung der Benzoyl-K-Säure wird durch Hochdruckflüssigkeitschromatographie bestimmt; es werden folgende Werte ermittelt:

| | |
|---|---|
| Benzoyl-K-Säure-dinatriumsalz | 85,1 Gew.% |
| Benzoyl-H-Säure | ∅ Gew.-% |
| K-Säure | ∅ Gew.-% |
| H-Säure | ∅ Gew.-% |
| iso-K-Säure | ∅ Gew.-% |
| Benzoesäure | ∅ Gew.-% |
| Wasser | 3,1 Gew.-% |
| Natriumchlorid | 0,2 Gew.-% |
| Natriumsulfat | 11,6 Gew.-% |

**Patentansprüche**

1. Verfahren zur Herstellung von 1-Benzoylami-no-8-hydroxy-naphthalin-4.6-disulfonsäure (Benzoyl-K-Säure) in Form ihrer Alkalisalze durch Umsetzung von 1-Amino-8-hydroxynaphthalin-4.6-disulfonsäure (K-Säure) mit Benzoylierungsmitteln, dadurch gekennzeichnet, dass man anstelle von reiner K-Säure Amino-naphthol-disulfonsäure-Isomerengemische verwendet, die über 40 Gew.-% K-Säure, bezogen auf die Gesamtmenge an diazotierbaren Verbindungen im Isomerengemisch, enthalten, bei Zugrundelegung eines Molekulargewichtes von 319 und in denen der Anteil der einzelnen anderen Isomeren an der Gesamtmenge der diazotierbaren Verbindungen im Isomerengemisch nicht mehr als 10 Gew.-% beträgt.

2. Verfahren gemäss Anspruch 1, dadurch gekennzeichnet, dass man Amino-naphthol-disulfonsäure-Isomerengemische verwendet, die 50 bis 90 Gew.-% K-Säure enthalten, bezogen auf die Gesamtmenge an diazotierbaren Verbindungen in den Isomerengemischen bei Zugrundelegung eines Molekulargewichtes von 319.

3. Verfahren gemäss Ansprüchen 1 und 2, dadurch gekennzeichnet, dass man Amino-naphthol-disulfonsäure-Isomerengemische verwendet, die 55 bis 75 Gew.-% K-Säure, 0 bis 10 Gew.-% H-Säure und 2 bis 10 Gew.-% Iso-K-Säure enthalten, Gew.-% bezogen auf die Gesamtmenge an diazotierbaren Verbindungen im Isomerengemisch.

4. Verfahren gemäss Ansprüchen 1 bis 3, dadurch gekennzeichnet, dass man als Amino-naph-thol-disulfonsäure-Isomerengemische solche ver-wendet, wie sie bei der alkalischen Druckhydrolyse von 1-Naphthylamin-4.6.8-trisulfonsäure (Melansäure) oder von Melansäure-Isomerengemischen anfallen, wobei man aus den bei der alkalischen Druck-hydrolyse von Melansäure-Isomerengemischen an-fallenden Amino-naphthol-disulfonsäure-Isomeren-gemischen, gegebenenfalls 1-Naphthylamin-8-hydroxy-naphthalin-3.6-disulfonsäure (H-Säure) durch Ansäuern ausgefällt und abgetrennt hat.

5. Verfahren gemäss Ansprüchen 1 bis 4, dadurch gekennzeichnet, dass man das Benzoylierungsmittel in einer solchen Menge einsetzt, dass 1,0 bis 1,7 Mol Benzoylierungsmittel auf 1 Mol diazotierbare Verbindung in den Amino-naphthol-disulfonsäure-Isomerengemischen entfallen.

6. Verfahren gemäss Ansprüchen 1 bis 5, dadurch gekennzeichnet, dass man das Benzoylierungsmittel in einer solchen Menge einsetzt, dass 1,1 bis 1,4 Mol Benzoylierungsmittel auf 1 Mol diazotierbare Verbindung in den Amino-naphthol-disulfonsäure-Isomerengemischen entfallen.

7. Verfahren gemäss Ansprüchen 1 bis 6, dadurch gekennzeichnet, dass man die Abscheidung der Benzoyl-K-Säure in Form ihrer Alkalisalze bei einem pH-Wert von 3 bis 6 durchführt.

8. Verfahren gemäss Ansprüchen 1 bis 7, dadurch gekennzeichnet, dass man die Isolierung der Benzoyl-K-Säure in Form ihrer Alkalisalze bei 0 bis 60°C vornimmt.

9. Verfahren gemäss Ansprüchen 1 bis 8, dadurch gekennzeichnet, dass man die Benzoyl-K-Säure in Form ihrer Alkalisalze aus Benzoylierungsgemischen ausfällt, deren Alkaliionen-Konzentration 2 bis 5 g Äquivalent/l Benzoylierungsgemisch beträgt.

**Claims**

1. Process for the preparation of 1-benzoylamino-8-hydroxynaphthalene-4,6-disulphonic acid (benzoyl-K acid) in the form of its alkali metal salts by reacting 1-amino-8-hydroxynaphthalene-4,6-disulphonic acid (K acid) with benzoylating agents, characterised in that mixtures of aminonaphtholdisulphonic acid isomers are used instead of pure K acid, which mixtures contain over 40% by weight of K acid, relative to the total amount of diazotisable compounds in the mixture of isomers, on the basis of a molecular weight of 319, and in which the proportion of each of the other isomers in the total amount of the diazotisable compounds in the mixture of isomers is no higher than 10% by weight.

2. Process according to claim 1, characterised in that mixtures of aminonaphtholdisulphonic acid isomers are used, which mixtures contain 50 to 90% by weight of K acid, relative to the total amount of diazotisable compounds in the mixtures of isomers, on the basis of a molecular weight of 319.

3. Process according to claims 1 and 2, characterised in that mixtures of aminonaphtholdisulphonic acid isomers are used, which mixtures contain 55 to 75% by weight of K acid, 0 to 10% by weight of H acid and 2 to 10% by weight of iso-K acid, % by weight being relative to the total amount of diazotisable compounds in the mixture of isomers.

4. Process according to claims 1 to 3, characterised in that those mixture of aminonaphtholdisulphonic acid isomers are used which are obtained in the alkaline hydrolysis under pressure of 1-naphthylamine-4,6,8-trisulphonic acid (melanic acid) or of mixtures of melanic acid isomers, 1-naphthylamine-8-hydroxynaphthalene-3,6-disulphonic acid (H acid) if appropriate having been precipitated by acidifying and removed from the mixtures of aminonaphtholdisulphonic acid isomers obtained in the alkaline hydrolysis under pressure of mixtures of melanic acid isomers.

5. Process according to claims 1 to 4, characterised in that the benzoylating agent is used in an amount which is such that 1.0 to 1.7 mols of benzoylating agent are used per 1 mol of diazotisable compound in the mixtures of aminonaphtholdisulphonic acid isomers.

6. Process according to claims 1 to 5, characterised in that the benzoylating agent is used in an amount which is such that 1.1 to 1.4 mols of benzoylating agent are used per 1 mol of diazotisable compound in the mixtures of aminonaphtholdisulphonic acid isomers.

7. Process according to claims 1 to 6, characterised in that the precipitation of benzoyl-K acid, in the form of its alkali metal salts, is carried out at a pH value of 3 to 6.

8. Process according to claims 1 to 7, characterised in that the isolation of benzoyl-K acid, in the form of its alkali metal salts, is carried out at 0 to 60°C.

9. Process according to claims 1 to 8, characterised in that the benzoyl-K acid in the form of its alkali metal salts, is precipitated from benzoylating mixtures having an alkali metal ion concentration of 2 to 5 g equivalents per litre of benzoylating mixture.

**Revendications**

1. Procédé de préparation de l'acide 1-benzoyl-amino-8-hydroxynaphtalène-4,6-disulfonique (acide benzoyl-K) à l'état de sels alcalins par réaction de l'acide 1-amino-8-hydroxynaphtalène-4,6-disulfonique (acide K) avec des agents benzoylants, caractérisé en ce que, au lieu d'utiliser de l'acide K pur, on utilise les mélanges d'acides aminonaphtol-disulfoniques isomères contenant plus de 40% en poids d'acide K, par rapport à la quantité totale des composés diazotables contenus dans le mélange des isomères, en se basant sur un poids moléculaire de 319, et dans lesquels la proportion des divers autres isomères par rapport à la quantité totale des composés diazotables contenus dans le mélange des isomères ne dépasse pas 10% en poids.

2. Procédé selon la revendication 1, caractérisé en ce que l'on utilise des mélanges d'acides aminonaphtol-disulfoniques isomères contenant 50 à 90% en poids d'acide K par rapport à la quantité totale des composés diazotables contenus dans les mélanges des isomères, en se basant sur un poids moléculaire de 319.

3. Procédé selon les revendications 1 et 2, caractérisé en ce que l'on utilise des mélanges d'acides aminonaphtol-disulfoniques isomères contenant 55 à 75% en poids d'acide K, 0 à 10% en poids d'acide H et 2 à 10% en poids d'acide iso-K, les pourcents en poids se rapportant à la quantité totale de composés diazotables contenus dans le mélange des isomères.

4. Procédé selon les revendications 1 à 3, caractérisé en ce que l'on utilise des mélanges d'acides aminonaphtol-disulfoniques isomères tels qu'obtenus à l'hydrolyse alcaline sous pression de l'acide 1-naphtylamine-4,6,8-trisulfonique (acide mélanique) ou de mélanges d'acides mélaniques isomères, en précipitant et en séparant éventuellement l'acide 1-naphtylamine-8-hydroxynaphtalène-3,6-disulfonique (acide H) par acidification du mélange des acides aminonaphtol-disulfoniques isomères obtenu à l'hydrolyse alcaline sous pression des mélanges d'acides mélaniques isomères.

5. Procédé selon les revendications 1 à 4, caractérisé en ce que l'on utilise l'agent benzoylant en quantité de 1,0 à 1,7 moles par mole de composés diazotables contenus dans les mélanges d'acides aminonaphtol-disulfoniques isomères.

6. Procédé selon les revendications 1 à 5, caractérisé en ce que l'on utilise l'agent benzoylant en quantité de 1,1 à 1,4 moles par mole de composés diazotables contenus dans les mélanges d'acides aminonaphtol-disulfoniques isomères.

7. Procédé selon les revendications 1 à 6, caractérisé en ce que l'on sépare l'acide benzoyl-K à l'état de sel alcalin à un pH de 3 à 6.

8. Procédé selon les revendications 1 à 7, caractérisé en ce que l'on isole l'acide benzoyl-K à l'état de sel alcalin à une température de 0 à 60°C.

9. Procédé selon les revendications 1 à 8, caractérisé en ce que l'on précipite l'acide benzoyl-K à l'état de sel alcalin à partir de mélanges de benzoylation dont la concentration en ions alcalins est de 2 à 5 équivalents-grammes par litre du mélange de benzoylation.